# EUROPÄISCHE PATENTSCHRIFT

(11) **EP 3 038 589 B1**
(45) Veröffentlichungstag und Bekanntmachung des Hinweises auf die Patenterteilung: **01.05.2019**
(21) Anmeldenummer: 14755089.1
(22) Anmeldetag: 25.08.2014
(51) Int. Cl.: A61K 8/25, A61K 8/31, A61K 8/34, A61Q 19/00, A61K 8/81, A61K 8/85, A61Q 19/10, A61K 8/92

(54) **EMULGATORFREIE, HAUTKONDITIONIERENDE, KOSMETISCHE ODER DERMATOLOGISCHE ZUBEREITUNG MIT PEELINGWIRKSTOFFEN**
EMULSIFIER-FREE, SKIN CONDITIONING, COSMETIC OR DERMATOLOGICAL PREPARATION WHICH CONTAINS PEELING ACTIVE INGREDIENTS
PRÉPARATION COSMÉTIQUE OU DERMATOLOGIQUE DE CONDITIONNEMENT DE LA PEAU SANS ÉMULSIFIANT ET COMPORTANT DES AGENTS EXFOLIANTS

(30) Priorität: 29.08.2013 DE 102013217242
(43) Veröffentlichungstag der Anmeldung: 06.07.2016
(73) Patentinhaber: Beiersdorf AG, 20253 Hamburg (DE)
(72) Erfinder: ARGEMBEAUX, Horst, 21465 Wentorf (DE)
(86) Internationale Anmeldenummer: PCT/EP2014/067975
(87) Internationale Veröffentlichungsnummer: WO 2015/028418

(56) Entgegenhaltungen:
- WO-A2-2009/135755
- WO-A2-2013/064391
- DE-A1-102006 029 879
- DE-A1-102011 013 342
- US-A1- 2003 211 061
- US-B1- 7 368 122

## Beschreibung

Die Erfindung umfasst eine emulgatorfreie, hautkonditionierende kosmetische oder dermatologische Zubereitung mit Peelingwirkstoffen. Die Zubereitung eignet sich für die Applikation auf nasser Haut und ermöglicht damit während des Duschens das Eincremen und gleichzeitiges Peeling.

Das Eincremen unter feuchten Bedingungen, Hautpflege unter der Dusche, wird zusammenfassend als Hautkonditionierung verstanden. Dies bedeutet u.a.:
1. Verwendung eines üblichen Duschproduktes zur Reinigung der Haut, Abspülung
2. Auftragen/Verteilung der erfindungsgemäßen Zubereitung auf nasser Haut
3. Erneutes Abduschen mit warmen oder kaltem Wasser
4. Abtrocknen der Haut

In der WO 2013064391 A2 werden kosmetische oder dermatologische Zubereitungen beschrieben, die ein Eincremen unter der Dusche ermöglichen.

Aufgrund von Zeitmangel wünschen sich viele Menschen eine möglichst effiziente Hautpflege und zeitgleichen Hautschutz.

Peeling (engl. to peel, "schälen, pellen") oder Schälkur ist eine kosmetische Behandlung, bei der oberflächliche Schichten der Haut flächig entfernt werden.

Beim oberflächlichen Peeling wird die oberste Hornschicht der Haut mechanisch oder chemisch entfernt. Diese Behandlung wird umgangssprachlich als Peeling bezeichnet, viele Anbieter bezeichnen diese Methode auch als Mikrodermabrasion.

Als Peelingmittel werden üblicherweise Polyethylen, Kochsalz, Meersalz, Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von Walnusschalen, Aprikosen-, Pfirsich- oder Mandelkernen den Zubereitungen zugesetzt.

Da die Peelingmittel feste partikuläre Substanzen sind, die abrasiv wirken, müssen diese zusammen mit der entfernten Haut nach der Anwendung abgespült werden. Dazu sind die bekannten Peelingzubereitungen zumeist mit waschaktiven Substanzen versehen, die ein Abspülen sicherstellen.

Nachteilig ist, dass die waschaktiven Substanzen die Haut austrocknen können und eine Hautschädigung die Folge sein kann.

Wünschenswert ist es eine Peelingzubereitung zur Verfügung zu stellen, die unter der Dusche angewendet werden kann und die aufgeführten Nachteile vermeidet.

Wünschenswert ist es eine Peelingzubereitung zur Verfügung zu stellen, die unter der Dusche angewendet werden kann und die aufgeführten Nachteile vermeidet.

Wünschenswert ist es eine Zubereitung zur Verfügung zu stellen, die einerseits hautverträglich gestaltet ist und andererseits die Möglichkeit eröffnet abrasiv wirkende Stoffe auf die Haut aufzubringen und dabei zeitsparend unter Dusche bzw. auf nasser Haut angewendet werden kann.

Die Erfindung ist eine emulgatorfreie kosmetische oder dermatologische Zubereitung umfassend ein oder mehrere Polyacrylsäurepolymere, ein oder mehrere C14-22 Fettalkohole sowie ein oder mehrere Wachse und/oder ein Kohlenwasserstoffgemisch sowie ein oder mehrere Peelingwirkstoffe mit einer Partikelgröße von 50 bis 500 µm.

Bevorzugt umfasst die Zubereitung Wachse, insbesondere Cera Microcristallina, im Bereich von mehr als 0,5 Gew.%, insbesondere mehr als 13 Gew.%, bezogen auf die Gesamtmasse der Zubereitung. Der bevorzugte Anteil bezieht sich sowohl auf einzelne Wachse als auch bevorzugt auf die Gesamtmenge an mehreren Wachsen.

Die Peelingwirkstoffe (Peelingkörper/ Peelingpartikel) werden bevorzugt gewählt aus der Gruppe PG-10 stearat, Polyethylen, Kochsalz, Meersalz, Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von z.B. Walnusschalen, Aprikosen-, Pfirsich- oder Mandelkernen. Bevorzugte Peelingmittel sind aus der Gruppe Himalayasalz, Kunstoffpartikel aus Viskose, Cellulose, Polypropylen, Polyester, Polyethylenterephthalat (PET), Polytetraflourethylen (PTFE), Aramid, Nylon, Kevlar, Polyvinylderivate, Polyurethane, Polylactid, Polyhydroxyalkanoat, Polycarbonat, Polystyrol, Celluloseester und/oder Polyethylen, sowie schwer- bzw. unlösliche Kristalle wie z.B. Calciumsulfat, Calciumcarbonat, verkapselte oder unverkapselte Kristalle wie z.B. Calciumchlorid, Kaliumchlorid, Magnesium-chlorid, Zucker, Silikate wie z.B. Seesand, hydrated Silica (Kieselsäure), Tonerde, Schlämme, geschrotete oder gemahlene Naturprodukte wie z.B. Weizen, Leinsamen, Reis, Mais, Mandeln, Nüsse, Nussschalen, Kürbiskerne, Kümmel, geschrotete oder gemahlene natürliche Schwämme wie z.B. Luffagurke, natürliche und synthetische Wachse wie z.B. Reiskleiewachs, Carnaubawachs, Jojobawachs, Bienenwachs, zu wählen.

Weiter bevorzugt können die Peelingmittel aus der Gruppe Rizinusöl (hydrogenated castor oil), Kieselsäure (hydrated Silica) und/oder Polymilchsäure (Polylactic acid, PLA), jeweils mit einer Partikelgröße von 50 bis 500 µm, gewählt werden.

Als bevorzugte Peelingkörper mit hydrogenated castor oil werden solche eingesetzt, die Rizinusöl, d.h. das aus Rizinus communis Samen gewonnene Öl, das anschließend zumindest teilweise hydriert wird, enthalten.

Die WO 2009135755 A offenbart eine emulgatorfreie in-shower Beispielzubereitung mit Acylates/C10-30 Alkyl Acrylat Crosspolymer, Octyldodecanol, Petrolatum und Mica.

Die US 2003211061 A1 beschreibt eine emulgatorfreie Beispielzubereitung zur Applikation auf nasser Haut umfassend Pemulen ® TR-2, Cetyl- und Stearylalkohol, Petrolatum und Bentonit. In der DE 102006029879 A1 werden emulgatorfreie Peeling Gele beschrieben.

Zu all den Mitteln, wie Mica und Bentonit, sind keine Partikelgrößen offenbart. Zudem ist nicht angegeben, ob diese Bestandteile partikuläre Eigenschaften haben und damit überhaupt als Peelingmittel wirken können.

Bevorzugt umfassen diese Peelingkörper mindestens 20% Rizinusöl, bevorzugt mindestens 50% Rizinusöl und am meisten bevorzugt mindestens 90 Gew.-% Rizinusöl, bezogen auf die Gesamtmasse des Peelingkörpers.

Der Grad der Hydrierung des Rizinusöls kann durch die Bestimmung der Jodzahl ermittelt werden. Besonders geeignet haben sich Rizinusöle mit einer Jodzahl von 0 bis 20 mg I₂/100g erwiesen.

Grundsätzlich können die Peelingkörper neben dem hydrierten Rizinusöl auch noch weitere Stoffe, insbesondere Wachse enthalten. Besonders geeignet sind Paraffinwachse, Carnaubawachse, Candelilawachse, Polyethylenwachse, oxidierte Polyethylenwachse oder Mischungen davon. Vorteilhaft sind solche Wachse zu wählen, die einen Schmelzbereich oberhalb von 40°C, bevorzugt oberhalb von 80°C aufweisen.

Hergestellt wird der Peelingkörper bevorzugt durch ein Verfahren, bei dem das hydrierte Rizinusöl, gegebenenfalls unter Zusatz weiterer Stoffe, insbesondere weiterer Wachse, geschmolzen wird und anschließend die Schmelze vertropft oder versprüht wird.

Grundsätzlich ist es auch möglich, die Peelingkörper auf Basis von hydriertem Rizinusöl mit Abrasivstoffen, beispielsweise mit Polyurethanreibkörpern zu mischen und dann in die erfindungsgemäßen Zubereitungen einzusetzen.

Erfindungsgemäß vorteilhaft ist die Wahl der Peelingwirkstoffe aus der Gruppe der lipophilen Verbindungen zu wählen.

Da die erfindungsgemäße Grundzubereitung aus überwiegend lipophilen Stoffen aufgebaut ist, kann so ein längerer Verbleib auf der Haut realisiert werden.

Der Anteil an einem oder mehreren Peelingwirkstoffen beträgt vorteilhaft bis zu 25 Gew.%, bezogen auf die Gesamtmasse der Zubereitung.

Erstaunlicherweise gelingt das Peeling mit der erfindungsgemäßen Zubereitung und es bleibt nach dem Abspülen und Abtrocknen auf der Haut ein pflegender Film auf der Haut zurück.

Die Anwendung der Kombination aus speziellen Fettalkoholen, Wachsen und vorteilhaft einem oder mehreren Filmbildnern führt nach dem Peelen zu einem Hautschutz- und/oder Hautpflege schon unter der Dusche.

Die erfindungsgemäßen Zubereitungen umfassen vorteilhaft ein oder mehrere Filmbildner.

Vorteilhaft werden diese Filmbildner gewählt aus der Gruppe Hexadecencopolymer, Trimethylsiloxysilicat, Polypropylsilsesquioxan, Polysilicone-25, Acrylatcopolymer, Polyurethan, Methacrylat, Polyglycerylstearat, Dilinoleat-Crosspolymer, Akyl-Acrylate/Methacrylic Acid Crosspolymer, IPDI Copolymer, insbesondere VP/Hexadecene Copolymer, Octyldodecyl Citrate Crosspolymer, Trimethylsiloxysilicat/ Polypropylsilsesquioxan, Polysilicone-25, Ammoniumacrylate - Copolymer, Acrylatcopolymer, Polyurethane-2 und Polymethylmethacrylat, Polyglycerylstearat/ Isostearatdilinoleat - Crosspolymer, Octadecen/MA Copolymer (und) Methylacetylricinoleat (und) Diisooctyladipat, Trimethylsiloxysilicat, Polyurethan-34, C8-22 Alkyl Acrylate/Methacrylic Acid Crosspolymer und/oder Castor Oil/IPDI Copolymer.

Der Anteil an Filmbildnern wird vorteilhaft im Bereich von 0,1 Gew.%, insbesondere 0,5 Gew.% bis 10 Gew.%., bezogen auf die Gesamtmasse der Zubereitung gewählt.

In einem Vergleich wird der sich bildende Schutzfilm auf der Haut untersucht, einmal nach der Anwendung eines rinse off Produktes mit integrierten Hautpflegeaspekten (Nivea creme soft shower gel) allein und einmal nach Anwendung des gleichen Produktes (Nivea creme soft shower gel) und anschließender Anwendung der erfindungsgemäßen Zubereitung. Nach beiden Anwendungen wird die Haut mit Wasser abgespült.

Die durchgeführten Messungen zur Rückfettung der Haut wurden per IR-Imaging gemacht. Die Messtechnik wird IR-ATR (InfraRed-Attenuated Total Reflectance) bezeichnet.

Es zeigte sich, dass erst nach der Anwendung der erfindungsgemäßen Zubereitung ein mittels der erwähnten Messtechnik sichtbarer Schutzfilm auf der Haut verbleibt und nachweisbar ist. Der Nachweis erfolgt über die Intensität der Kohlenwasserstoff-IR -Banden (CH-IR bands).

Der erfindungsgemäße Schutzfilm umfasst einen auf der Haut sich bildenden Film.

Die erfindungsgemäße Hautkonditionierung umfasst das Eincremen unter feuchten Bedingungen, insbesondere der Hautpflege unter der Dusche, wobei ein Schutzfilm auch nach dem Abspülen auf der Haut verbleibt. Der Schutzfilm ist mittels IR-ATR Messtechnik nachweisbar und weist idealerweise eine Dicke von mindestens 1 µm bis zu 10 µm auf.

Insbesondere ist die erfindungsgemäße Hautkonditionierung dadurch gekennzeichnet, dass im Schutzfilm auf der Haut ein oder mehrere Wirkstoffe, ein oder mehrere Lipide und ein oder mehrere Hautbefeuchtungsmittel umfasst und keine die Hautbarriere schädigenden Stoffe, insbesondere keine Emulgatoren und/oder Tenside, enthalten sind.

Erst dieser auf der Haut verbleibende Film ermöglicht die Aufbringung und auch den Verbleib von Wirkstoffe auf der Haut.

Bei der erfindungsgemäßen Zubereitung werden ein oder mehrere Fettalkohole und mindestens ein zusätzliches Wachs und/oder Gemisch aus flüssigen und festen Kohlenwasserstoffen, mit einem Schmelzbereich von 5°C bis 75°C, bevorzugt bis 55°C (nach DSC), zusammen gegeben, insbesondere aufgeschmolzen.

D.h. entweder ist neben mindestens einem Fettalkohol mindestens ein Wachs enthalten oder neben Fettalkohol ist mindestens ein Gemisch aus flüssigen und festen Kohlenwasserstoffen enthalten. Idealerweise sind Fettalkohol, Wachs und ein Gemisch aus Kohlenwasserstoffen enthalten.

Vorteilhaft sind als Fettalkohole Myristyl-, Cetearyl- und/oder Stearylalkohole zu wählen, als Wachse Cera Microcristallina, Coco-Glycerid, C18-36 Acid Triglycerid, Synthetic Wax, Cera Alba, Paraffin, Copernicia Cerifera Cera, C18-38 Alkyl Hydroxystearoylstearat, Butyrospermum Parkii Butter, Olus oil, C20-40 Alkohol und/oder Bienenwachs und als Kohlenwasserstoffgemisch Paraffinum Liquidum zu wählen.

Bevorzugt ist Cera Microcristallina.

Bevorzugte Einsatzkonzentrationen des oder der Wachse liegt im Bereich von 0,5 bis 20 Gew.%, bezogen auf die Gesamtmasse der Zubereitung.

Die Fettalkohole, Wachse und Kohlenwasserstoffgemische weisen dabei vorteilhaft alle einen Schmelzbereich von 5°C bis 75°C, bevorzugt bis 55°C (nach DSC) auf.

DSC (Differential Scanning Calorimetry) ist ein thermisches Verfahren zur Messung von abgegebener/aufgenommener Wärmemenge einer Probe bei isothermer Arbeitsweise, Aufheizung oder Abkühlung (siehe DIN 53765, DIN 51007, ASTM E 474, ASTM D 3418). DSC ist eine vergleichende Messmethode, die die Bestimmung von Wärmemengen physikalischer und chemischer Prozesse ermöglicht. Wenn ein Material seinen physikalischen Zustand ändert, wie z.B. Schmelzen oder Umwandlung einer Kristallform in eine andere oder wenn es chemisch reagiert, wird Wärme dabei aufgenommen oder abgegeben. Diese Wärmemengen sind mit Hilfe der DSC quantitativ messbar. Die Methode verläuft zyklisch, so dass nach der ersten Aufheizkurve ein definiertes Abkühlen stattfindet und anschließend die Probe noch einmal im angegebenen Temperaturbereich aufgeheizt wird. Man erhält somit zweierlei Informationen: In der ersten Aufheizkurve sind alle thermische Effekte inklusiv Vorgeschichte erkennbar. In der zweiten Aufheizkurve ist die Vorgeschichte eliminiert worden und das reine thermische Verhalten der Probe unter definierten Abkühlbedingungen ist auswertbar. Der Schmelzbereich der Fettalkohole, Wachse bzw. Kohlenwasserstoffe zwischen 4,5°C und 75°C nach DSC ist der in der ersten Aufheizkurve ermittelte Bereich.

Als Wachse können erfindungsgemäß auch Fette und fettähnlichen Substanzen mit wachsartiger Konsistenz eingesetzt werden. Hierzu gehören u.a. Fette (Triglyceride), Mono und Diglyceride, natürliche und synthetische Wachse, Fett-und Wachsalkohole, Ester von Fettalkoholen und Fettsäuren sowie Fettsäureamide oder beliebige Gemische dieser Substanz.

Besonders bevorzugt werden die Wachse aus der Gruppe der Fette , insbesondere aus der Gruppe natürliche Wachse: Shorea Stenoptera Seed Butter, Hydrogenated Vegetable Oil, Hydrogenated Coco-Glycerides, Butyrospermum Parkii Butter, Theobroma Cacao (Cocoa) Seed Butter, Mango Butter, Hydrogenated Palm Kernel Glycerides, Hydrogenated Palm Glycerides, Sunflower Seed Wax, Soybean Glycerides, Butyrospermum Parkii Unsaponifiables, , Wollwachs , Cera Alba, Beeswax, Zuckerrohrwachs, Cera Carnauba, Candelillawachs, Japanwachs, Hydrogenated Rapeseed Oil, Shellac Wax, Hydrogenated Lecithin, Hydrogenated Soybean Oil,
aus der Gruppe synthetischer Wachse, insbesondere aus:
Cera Microcristallina, Synthetic Beeswax, Synthetic Wax, Polyethylene, Paraffin Wax, Ceresin, Ozokerite
aus der Gruppe der Fettsäuren, insbesondere aus:
Palmitic Acid, Stearic Acid,
aus der Gruppe der Ester aus Fettsäuren, insbesondere aus
Cetearyl Nonanoate, Methyl Palmitate, Glyceryl Tribehenate, Glyceryl Laurate, Glyceryl Stearate, Cetyl Palmitate; Shea Butter Oleyl Esters, PEG-8 Beeswax
gewählt.

Es ist erfindungsgemäß zu unterscheiden zwischen den als Peelingmittel bevorzugt zu wählenden Wachsen und den Wachsen der Zubereitung.

Als Fettalkohole werden bevorzugt C14 bis C22 Fettalkohole verwendet. Bevorzugt werden die Fettalkohole gewählt aus der Gruppe linearen Fettalkohole, insbesondere Myristylalkohol (C₁₄H₃₀O), Cetylalkohol (oder Palmitylalkohol) (C₁₆H₃₄O), Stearylalkohol (oder Octadecylalkohol) (C₁₈H₃₈O) sowie Cetylstearylalkohol (Cetearylalkohol),Behenylalkohol, Lanolin Alcohol, ein Gemisch der Alkohole Cetylalkohol (Hexadecanol) und Stearylalkohol (Octadecanol).

Der Anteil an C14-22 Fettalkoholen insgesamt beträgt vorteilhaft 0,5 bis 14 Gew.%, insbesondere 7 bis 9 Gew.%, bzw. insbesondere 0,5-5% bezogen auf die Gesamtmasse der Zubereitung.

Als Kohlenwasserstoffgemische werden bevorzugt Kohlenwasserstoffgele bzw. Mischungen aus flüssigen und festen Paraffinkohlenwasserstoffen verwendet. Bevorzugt liegt der Gehalt an festen Kohlenwasserstoffen im Kohlenwasserstoffgemisch zwischen 1 und 50%, besonders bevorzugt zwischen 10 und 30%. Vorteilhaft ist die Verwendung von Kohlenwasserstoffgemischen, die Fransenmizellen bzw. parakristalline Strukturen ausbilden. Der Anteil des Kohlenwasserstoffgemisch insgesamt beträgt vorteilhaft 1 bis 50 Gew.%, insbesondere 20 bis 30 Gew.%, bezogen auf die Gesamtmasse der Zubereitung.

Fettalkohol, insbesondere zwei oder drei Fettalkohole, sind erfindungsgemäß zwingend in der Zubereitung enthalten. Zusätzlich wird ein oder mehrere Wachse der Zubereitung hinzugefügt. Bevorzugt kann anstelle des Wachses auch ein Kohlenwasserstoffgemisch aus bei Raumtemperatur flüssigen und festen Kohlenwasserstoffen zugesetzt werden.

Idealerweise umfasst die Zubereitung alle drei Bausteine, Fettalkohole, Wachse und Kohlenwasserstoffgemisch.

Der erfindungsgemäßen Herstellung und Zubereitung können dann dem Fachmann bekannte kosmetische oder dermatologische Stoffe zugegeben werden, wobei deren Zugabe nicht die hautkonditionierenden Eigenschaften und das Peeling der erhältlichen Zubereitung beeinträchtigen darf.

Vorteilhaft werden den Zubereitungen Verdicker, Füllstoffe und Neutralisierungsmittel zugesetzt.

Verdicker sind zur Stabilisierung des Systems vorteilhaft geeignet und verstärken die hautkonditionierenden Eigenschaften und das spezielle Hautgefühl der erfindungsgemäßen Zubereitungen.

Als Füllstoff wird bevorzugt Aluminium Starch Octenylsuccinat zugesetzt, was ebenfalls zu einer Optimierung des Hautgefühls führt indem der Hautschutzfilm etwas samtiger wirkt.

Als Neutralisierungsmittel wird vorteilhaft Natronlauge zugegeben, damit die Verdicker ihr Gelnetzwerk ausbilden können und ein stabiles System entsteht.

Die erfindungsgemäße Zubereitung ist emulgatorfrei. D.h. die ggf. als emulgierend wirkenden Polyacrylsäurepolymere oder Peelingmittel werden erfindungsgemäß nicht als Emulgatoren verstanden.

Anders ausgedrückt, neben den Polyacrylsäurepolymeren und/oder Peelingmittel werden der Zubereitung keine weiteren Emulgatoren zugesetzt.

Emulgatorfrei umfasst auch ein Mindestgehalt an zusätzlichen Emulgatoren von weniger als 1 Gew.%, bezogen auf die Gesamtmasse der Zubereitung, die beispielsweise durch Verunreinigungen oder Einschleppungen enthalten sein können. Der Einfluss auf die Produktperfomance ist in diesen Mengenbereichen ggf. unbeachtlich.

Bevorzugt wird allerdings ein Gehalt an Emulgatoren von 0 Gew.%.

Als Polyacrylsäurepolymere werden die in der Kosmetik bekannten Polymere der Acryl- und/oder Methacrylsäure sowie Acrylat-Crosspolymere verstanden.

Vorzugsweise sind dies Polymere (Makromoleküle) mit hohem Molekulargewicht (> 1 Mg/mol), welche aus einem Gerüst aus Polyacrylsäure und geringen Mengen an Polyalkenylether-Quervernetzungen bestehen. Sie werden auch als Carbomere bezeichnet. Diese wasserlöslichen oder dispergierbaren Polymere können in der Flüssigkeit, in der sie gelöst oder dispergiert sind, eine bedeutende Viskositätserhöhung hervorrufen. Dies wird durch die Bildung von Carbomer-Mikrogelen im Wasser hervorgerufen.

Besonders bevorzugte Polyacrylsäurepolymere sind neben den Carbomeren diejenigen Acrylat-Crosspolymere, die eine polymere Emulgatorwirkung ausüben.

Polymere Emulgatoren sind hauptsächlich Polyacrylsäurepolymere mit hohem Molekulargewicht. Diese emulgierend wirkenden Polyacrylsäurepolymere haben einen kleinen lipophilen Anteil zusätzlich zum hydrophilen Hauptteil. Ganz besonders bevorzugt im Sinne der vorliegenden Erfindung sind Acrylat-Crosspolymere, welche die INCI Bezeichnung "Acrylates/C10-30 Alkyl Acrylate Crosspolymer" haben und unter den Handelsbezeichnungen PemulenTR-1 und Pemulen TR-2 sowie Carbopol 1342, Carbopol 1382 und Carbopol ETD 2020 von der Firma NOVEON erhältlich sind.

Besonders bevorzugt werden die Polyacrylsäurepolymere gewählt aus der Gruppe der Acrylates/C10-30 Alkyl Acrylate Crosspolymere und/oder Carbomere. Insbesondere bevorzugt sind Acrylates/C10-30 Alkyl Acrylate Crosspolymer Pemulen® TR-1, z.B. von Lubrizol und Carbopol® 3128 von Lubrizol.

Erfindungsgemäß ist hierbei eine spezifische Kombination aus Polyacrylsäurepolymeren mit emulgierender Wirkung, wie das Pemulen TR-1 mit anderen Polyacrylsäurepolymeren, wie Carbopol 3128, die die sensorischen Eigenschaften verbessern und die Stabilität der Zubereitung, insbesondere bei höheren Temperaturen, und eine Verbindung mit freiem Wasser gewährleisten.

Besonders bevorzugt ist hierbei eine Kombination aus drei Polyacrylsäurepolymeren, wobei ein Polyacrylsäurepolymer eine emulgierende Wirkung aufweist, wie z.B. das Pemulen TR-1 oder Pemulen TR-2, mit anderen Polyacrylsäurepolymeren, die die sensorischen Eigenschaften verbessern und die Stabilität der Zubereitung, insbesondere bei höheren Temperaturen, gewährleisten (z.B. Carbopol 3128) und einem Polyacrylsäurepolymer, das die sensorischen Eigenschaften bei Aufnahme von freiem Wasser verbessern (z.B. Carbopol 981).

Vorteilhaft umfasst die erfindungsgemäße Zubereitung daher bevorzugt mindestens drei Polyacrylsäurepolymere, insbesondere drei Polyacrylsäurepolymere, die sich in ihren Eigenschaften unterscheiden.

Der Anteil an Polyacrylsäurepolymeren insgesamt beträgt vorzugsweise 0,05 bis 2 Gew.%, insbesondere 0,2 bis 1 Gew.%, bezogen auf die Gesamtmasse der Zubereitung.

Vorteilhaft zeigte sich die Kombination mindestens zweier Polyacrylsäurepolymere mit mindestens zwei C14-22 Fettalkoholen zur verbesserten Stabilisierung der Zubereitung und vor allem das Hautgefühl bei der Anwendung auf feuchter/nasser Haut ist nicht unangenehm, nicht wachsig, stumpf oder quietschend.

Erfindungsgemäß sind zwei Polyacrylsäurepolymer bzw. drei Polyacrylsäurepolymere so zu verstehen, dass jeweils ein Polyacrylsäurepolymer sich von den jeweils anderen in zumindest einer Eigenschaft unterscheidet. Die Stoffgruppe Acrylates/C10-30 Alkyl Acrylate Crosspolymere umfasst beispielsweise die Handelsprodukte Pemulen TR-1 bzw. TR-2.

Carbomere werden beispielsweise in die Typen A, B und C unterschieden. Unterschiede sind hierin beispielsweise deren Gele mit unterschiedlichen Viskositäten (United States Pharmacopoeia, USP).

Erfindungswesentlich und damit bevorzugt ist zudem ein Anteil an Wachsen oder bevorzugt eine Mischung aus flüssigen und festen Kohlenwasserstoffen mit einem Schmelzbereich von 4,5 bis 75°C, insbesondere bis 55°C nach DSC.

Als Öle können optional vorteilhaft unpolare bis mittelpolare Lipide in den erfindungsgemäßen Zubereitungen zugesetzt werden. Ansonsten ist die Stabilität aufgrund der Emulgatorfreiheit schwieriger einzustellen.

Im Rahmen der vorliegenden Offenbarung wird als Oberbegriff für Fette, Öle, Wachse und dergleichen der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität. Es wird vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Die erfindungsgemäße Zubereitung ermöglicht erstmals die Anwendung des Peelings und gleichzeitigem Pflegens unter der Dusche sowie ggf. die Aufbringung von Wirkstoffen schon während des Duschvorgangs.

Die erfindungsgemäßen Zubereitungen werden vorteilhaft nur mit Konservierungsmitteln formuliert, die eine Wasserlöslichkeit von mehr als 0,75% bei 20°C aufweisen. Aufgrund des Fehlens von Emulgatoren kann es ansonsten zu Destabilisierungen und zur Auskristallisierung kommen.

Die erfindungsgemäßen Zubereitungen sind weiterhin bevorzugt auch frei von Tensiden.

Tenside sind Substanzen, die die Oberflächenspannung einer Flüssigkeit oder die Grenzflächenspannung zwischen zwei Phasen herabsetzen und die Bildung von Dispersionen ermöglichen oder unterstützen. Tenside bewirken, dass zwei eigentlich nicht miteinander mischbare Flüssigkeiten, wie zum Beispiel Öl und Wasser, dispergiert werden können.

Des Weiteren werden Tenside als amphiphile Stoffe beschrieben, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im Allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quatären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung.

Bekannt sind des Weiteren waschaktive Substanzen, wie beispielsweise kationische Tenside insbesondere quartäre Ammoniumverbindungen. Eine waschaktive Substanz findet in Waschmitteln, Spülmitteln, Shampoos, Duschgels Verwendung und bezeichnet den Anteil der Formulierung, der die Wasch- oder Reinigungsleistung beeinflusst. Waschaktive Substanzen erhöhen die "Löslichkeit" von Fett- und Schmutzpartikeln in Wasser, die in der Wäsche oder am Körper haften. Sie können natürlichen oder synthetischen Ursprungs sein. Sie werden nach der Art ihrer Ladung in anionisch, kationisch, ampholytisch oder nichtionisch unterschieden.

Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert" werden. Emulgatoren haben primär keinen waschaktiven, tensidischen Charakter.

Emulgatoren und Tenside können die Barriereschicht der Haut schädigen. Den Zubereitungen werden daher weder Emulgatoren noch Tenside zugesetzt, also vorteilhaft wird auf den Zusatz zusätzlicher waschaktiver Substanzen verzichtet.

Vorteilhaft sind den Zubereitungen Hautbefeuchtungsmittel, Moisturizer, zugesetzt.

Die Zubereitungen werden auf nasser Haut verwendet und insbesondere auch zur Rasur.

Die erfindungsgemäße Zubereitung kann zur Hautkonditionierung verwendet werden.

Sie ermöglicht die Erzeugung eines Hautschutzfilms nach Applikation der Zubereitung auf der Haut und anschließendem Abspülen mit Wasser.

Der sich bildende Schutzfilm ist idealerweise mind. 1 µm dick (gemessen nach IR-ATR Messtechnik) und/oder umfasst keine hautbarriereschädigende Stoffe, insbesondere keine Emulgatoren, Tenside, PEG's und/oder halogenorganische Verbindungen.

Des Weiteren können kosmetische Wirkstoffe in den erfindungsgemäßen Zubereitungen enthalten sein.

Bevorzugte Ausführungsformen der erfindungsgemäßen Zubereitungen umfassen ein oder mehrere Wachse, ein Kohlenwasserstoffgemisch und ein Wirkstoff aus der Gruppe der Hautbefeuchtungsmittel, wobei als Fettalkohole Myristyl-, Cetearyl- und/oder Stearylalkohole, als Wachs Cera Microcristallina und als Kohlenwasserstoffgemisch Paraffinum Liquidum gewählt wird.

Vorteilhaft ist in dieser Zubereitung als Hautbefeuchtungswirkstoff Glycerin, sowie Ubichinon Wirkstoff Q10 zur Hautpflege und ein kühlender Wirkstoff wie Menthol zu wählen.

Diese Dreierkombination an Wirkstoffen in Kombination mit einem Peelingmittel verursacht ein angenehmes Hautgefühl und -pflege sowohl während des Duschens als auch eine langanhaltende Hautpflege.

Bei Einschränkungen auf bevorzugt genannte Stoffe, seien es Lipide, Wachse, Peelingmittel, Wirkstoffe oder Filmbildner oder weitere bevorzugt gennannte Bestandteile, so beziehen sich deren bevorzugten Anteilsbereiche dann auch auf die dann ausgewählten Einzelbestandteile. Die anderen, die durch die Einschränkung ausgeschlossenen Bestandteile, zählen dann nicht mehr zu den aufgeführten Anteilsbereichen hinzu.

Nachfolgende Beispiele veranschaulichen die erfindungsgemäße Herstellung um erfindungsgemäße Zubereitungen zu erhalten.

Die Zahlenwerte sind Gewichtsanteile, bezogen auf die Gesamtmasse der Zubereitung.

**Beispiele**

| Inhaltststoff | 1 | 2 | 3 | 4 | 5 |
|---|---|---|---|---|---|
| Cera Microcristallina | 25,000 | 16,5000 | 16,5000 | 35,000 | 45,000 |
| Paraffinum Liquidum | | 8,5000 | 8,5000 | | |
| Myristylalkohol | 1,0000 | 1,0000 | 1,0000 | 2,0000 | |
| Cetearylalkohol | 5,0000 | 5,0000 | 5,0000 | 4,0000 | 4,0000 |
| Stearylalkohol | 2,0000 | 2,0000 | 2,0000 | 3,0000 | 3,0000 |
| Hydrierte Kokosglyceride | 3,0000 | 3,0000 | 3,0000 | 2,0000 | 2,0000 |
| Mandelöl | | | 0,3500 | | 0,7000 |
| Aluminumstärkeoctenylsuccinat | 1,0000 | 1,0000 | 1,0000 | 1,0000 | 1,0000 |
| Parfum | 0,8000 | 0,7000 | 1,0000 | 0,7000 | 0,7000 |
| Glycerin | 5,1000 | 5,1000 | 5,1000 | 15,100 | 10,100 |
| Natriumhydroxidlösung 45%ig | 0,1600 | 0,1600 | 0,1600 | 0,1600 | 0,1600 |
| Phenoxyethanol | 0,5000 | 0,5000 | 0,5000 | 0,4000 | 0,4000 |
| Methylisothiazolinone | 0,0900 | 0,0900 | 0,0900 | 0,0800 | 0,0800 |
| Acrylates/C10-30 Alkyl Acrylate Crosspolymer (Carbopol 3128) | 0,1000 | 0,1000 | 0,1000 | 0,1200 | |
| Carbomer (Carbopol 981) | 0,0200 | 0,0200 | 0,0200 | 0,0200 | 0,0200 |
| Acrylates/C10-30 Alkyl Acrylate Crosspolymer (Pemulen TR-1) | 0,1000 | 0,1000 | 0,1000 | 0,1200 | 0,1400 |
| Q10 | | | 0,0045 | | |
| Menthol | | | 0,3000 | | |
| Meersalz | 0,0100 | 0,0100 | | 0,0100 | 0,0500 |
| hydrogenated castor oil (50%) in Paraffinwachs | 10,0000 | | | | 25,000 |
| PLA | 0,5000 | | | 1,600 | |
| Kieselsäure | | 2,450 | 0,800 | | |
| Wasser | ad 100 | ad 100 | ad 100 | ad 100 | ad 100 |

## Patentansprüche

1. Emulgatorfreie kosmetische oder dermatologische Zubereitung umfassend ein oder mehrere Polyacrylsäurepolymere, ein oder mehrere C14-22 Fettalkohole und zusätzlich ein oder mehrere Wachse und/oder ein Kohlenwasserstoffgemisch **dadurch gekennzeichnet, dass** ein oder mehrere Peelingwirkstoffe enthalten sind und die Partikelgröße der Peelingwirkstoffe im Bereich von 50 bis 500 µm gewählt werden.

2. Zubereitung nach Anspruch 1 **dadurch gekennzeichnet, dass** der oder die Wachse zu einem Anteil von mehr als 0,5 Gew.%, bezogen auf die Gesamtmasse der Zubereitung, gewählt werden.

3. Zubereitung nach einem der vorstehenden Ansprüche **dadurch gekennzeichnet, dass** als Fettalkohole Myristyl-, Cetearyl- und/oder Stearylalkohole gewählt werden.

4. Zubereitung nach einem der vorstehenden Ansprüche **dadurch gekennzeichnet, dass** als Wachs Cera Microcristallina, Coco-Glycerid, C18-36 Acid Triglycerid, Synthetic Wax, Cera Alba, Paraffin, Copernicia Cerifera Cera , C18-38 Alkyl Hydroxystearoylstearat, Butyrospermum Parkii Butter, Olus oil, C20-40 Alkohol und/oder Bienenwachs gewählt werden

5. Zubereitung nach einem der vorstehenden Ansprüche **dadurch gekennzeichnet, dass** ein oder mehrere Filmbildner enthalten sind.

6. Zubereitung nach Anspruch 5 **dadurch gekennzeichnet, dass** als Filmbildner Hexadecencopolymer, Trimethylsiloxysilicat, Polypropylsilsesquioxan, Polysilicone-25, Acrylatcopolymer,Polyurethan, Methacrylat, Polyglycerylstearat, Dilinoleat-Crosspolymer, Akyl-Acrylate/Methacrylic Acid Crosspolymer, IPDI Copolymer, insbesondere VP/Hexadecene Copolymer, Octyldodecyl Citrate Crosspolymer, Trimethylsiloxysilicat/ Polypropylsilsesquioxan, Polysilicone-25, Ammoniumacrylate - Copolymer, Acrylatcopolymer, Polyurethane-2 und Polymethylmethacrylat, Polyglycerylstearat/ Isostearatdilinoleat - Crosspolymer, Octadecen/MA Copolymer (und) Methylacetylricinoleat (und) Diisooctyladipat, Trimethylsiloxysilicat, Polyurethan-34, C8-22 Alkyl Acrylate/Methacrylic Acid Crosspolymer und/oder Castor Oil/IPDI Copolymer gewählt werden.

7. Zubereitung nach Anspruch 5 oder 6 **dadurch gekennzeichnet, dass** als Filmbildner Castor Oil/IPDI Copolymer, Trimethylsiloxysilicat/Polypropylsilsesquioxan und/oder Acrylatcopolymer gewählt werden

8. Zubereitung nach Anspruch 5, 6 oder 7 **dadurch gekennzeichnet, dass** der oder die Filmbildner zu einem Anteil von 0,1 - 10 Gew.%, bezogen auf die Gesamtmasse der Zubereitung, gewählt werden.

9. Zubereitung nach einem der vorstehenden Ansprüche **dadurch gekennzeichnet, dass** als Peelingwirkstoffe ein oder mehrere Stoffe aus der Gruppe der Rizinusöl, Kieselsäure und/oder Polymilchsäure gewählt werden.

10. Zubereitung nach einem der vorstehenden Ansprüche **dadurch gekennzeichnet, dass** ein oder mehrere Hautbefeuchtungsmittel enthalten sind.

11. Verwendung der Zubereitung nach einem der vorstehenden Ansprüche auf nasser Haut.

## Claims

1. Emulsifier-free cosmetic or dermatological preparation comprising one or more polyacrylic acid polymers, one or more C14-22 fatty alcohols and additionally one or more waxes and/or a hydrocarbon mixture, **characterized in that** one or more peeling active ingredients are present and the particle size of the peeling active ingredients is selected in the range from 50 to 500 µm.

2. Preparation according to Claim 1, **characterized in that** the wax or the waxes are chosen to a fraction of more than 0.5% by weight, based on the total mass of the preparation.

3. Preparation according to any one of the preceding claims, **characterized in that** the fatty alcohols selected are myristyl, cetearyl and/or stearyl alcohols.

4. Preparation according to any one of the preceding claims, **characterized in that** the wax selected is Cera Microcristallina, coco glyceride, C18-36 acid triglyceride, synthetic wax, Cera Alba, paraffin, Copernicia Cerifera Cera, C18-38 alkyl hydroxystearoylstearate, Butyrospermum Parkii butter, olus oil, C20-40 alcohol and/or beeswax.

5. Preparation according to any one of the preceding claims, **characterized in that** one or more film formers are present.

6. Preparation according to Claim 5, **characterized in that** the film formers selected are hexadecene copolymer, trimethylsiloxysilicate, polypropylsilsesquioxane, polysilicone-25, acrylate copolymer, polyurethane, methacrylate, polyglyceryl stearate, dilinoleate crosspolymer, alkyl acrylate/methacrylic acid crosspolymer, IPDI copolymer, in particular VP/hexadecene copolymer, octyldodecyl citrate crosspolymer, trimethylsiloxysilicate/polypropylsilsesquioxane, polysilicone-25, ammonium acrylate copolymer, acrylate copolymer, polyurethane-2 and polymethyl methacrylate, polyglyceryl stearate/isostearate dilinoleate crosspolymer, octadecene/MA copolymer (and) methyl acetylricinoleate (and) diisooctyl adipate, trimethylsiloxysilicate, polyurethane-34, C8-22 alkyl acrylate/methacrylic acid crosspolymer and/or castor oil/IPDI copolymer.

7. Preparation according to Claim 5 or 6, **characterized in that** the film formers selected are castor oil/IPDI copolymer, trimethylsiloxysilicate/polypropylsilsesquioxane and/or acrylate copolymer.

8. Preparation according to Claim 5, 6 or 7, **characterized in that** the film former or the film formers are chosen to a fraction of 0.1-10% by weight, based on the total mass of the preparation.

9. Preparation according to any one of the preceding claims, **characterized in that** one or more ingredients are selected from the group of castor oil, silicic acid and/or polylactic acid as peeling active ingredients.

10. Preparation according to any one of the preceding claims, **characterized in that** one or more skin-wetting agents are present.

11. Use of the preparation according to any one of the preceding claims on wet skin.

## Revendications

1. Préparation cosmétique ou dermatologique exempte d'émulsifiants, comprenant un ou plusieurs polymères de type poly(acide acrylique), un ou plusieurs alcools gras en C₁₄₋₂₂ et en outre une ou plusieurs cires et/ou un mélange d'hydrocarbures, **caractérisée en ce qu'**un ou plusieurs principes actifs de peeling sont contenus et la grosseur de particule des principes actifs de peeling est choisie dans la plage de 50 à 500 µm.

2. Préparation selon la revendication 1, **caractérisée en ce que** la ou les cires sont choisies en une proportion supérieure à 0,5% en poids, par rapport à la masse totale de la préparation.

3. Préparation selon l'une quelconque des revendications précédentes, **caractérisée en ce qu'**on choisit, comme alcools gras, l'alcool myristylique, l'alcool cétéarylique et/ou stéarylique.

4. Préparation selon l'une quelconque des revendications précédentes, **caractérisée en ce qu'**on choisit, comme cire, Cera Microcristallina, un glycéride de coco, un triglycéride d'acide en C₁₈₋₃₆, une cire synthétique, Cera Alba, de la paraffine, Copernicia Cerifera Cera, un hydroxystéaroylstéarate de C₁₈₋₃₈-alkyle, le beurre de karité, une huile végétale (Olus oil), un alcool en C₂₀₋₄₀ et/ou la cire d'abeille.

5. Préparation selon l'une quelconque des revendications précédentes, **caractérisée en ce qu'**un ou plusieurs agents filmogènes sont contenus.

6. Préparation selon la revendication 5, **caractérisée en ce qu'**on choisit, comme agent filmogène, un copolymère d'hexadécène, un siloxysilicate de triméthyle, un polypropylsilsesquioxane, le polysilicone-25, un copolymère d'acrylate, le polyuréthane, un méthacrylate, un poly(stéarate de glycéryle), un polymère réticulé de dilinoléate, un polymère réticulé d'acrylate d'alkyle /acide méthacrylique, un copolymère d'IPDI, en particulier un copolymère de VP/hexadécène, un polymère réticulé de citrate d'octyldodécyle, un siloxysilicate de triméthyl/polypropylsilsesquioxane, le polysilicone-25, un copolymère d'acrylate d'ammonium, un copolymère d'acrylate, le polyuréthane-2 et le poly(méthacrylate de méthyle), un polymère réticulé de poly(stéarate de glycéryle/isostéarate-dilinoléate, un copolymère d'octadécène/MA (et) le ricinoléate de méthyle et d'acétyle (et) l'adipate de diisooctyle, le siloxysilicate de triméthyle, le polyuréthane-34, un polymère réticulé d'acrylate de C₈₋₂₂-alkyle/acide méthacrylique et/ou un copolymère d'huile de ricin/IPDI.

7. Préparation 1 selon la revendication 5 ou 6, **caractérisée en ce qu'**on choisit, comme agent filmogène, un copolymère d'huile de ricin/IPDI, le siloxysilicate de triméthyle/polypropylsilsesquioxane et/ou un copolymère d'acrylate.

8. Préparation selon la revendication 5, 6 ou 7, **caractérisée en ce que** le ou les agents filmogènes sont choisis en une proportion de 0,1-10% en poids, par rapport à la masse totale de la préparation.

9. Préparation selon l'une quelconque des revendications précédentes, **caractérisée** en' ce qu'on choisit, comme ingrédients actifs de peeling, une ou plusieurs substances dans le groupe formé par l'huile de ricin, la silice et/ou le poly(acide lactique).

10. Préparation selon l'une quelconque des revendications précédentes, **caractérisée en ce qu'**un ou plusieurs agents d'hydratation de la peau sont contenus.

11. Utilisation de la préparation selon l'une quelconque des revendications précédentes sur une peau humide.
